# FASCICULE DE BREVET EUROPEEN

(11) **EP 0 483 320 B1**
(45) Date de publication et mention de la délivrance du brevet: **31.08.1994**
(21) Numéro de dépôt: 91909171.0
(22) Date de dépôt: 16.05.1991
(51) Int. Cl.: A61K 9/20, A61K 47/38

(54) **COMPOSITIONS PHARMACEUTIQUES DU TYPE A LIBERATION PROLONGEE DESTINEES A L'ADMINISTRATION PAR VOIE ORALE ET LEUR PROCEDE DE PREPARATION**
PHARMAZEUTISCHE ZUSAMMENSETZUNGEN MIT VERZÖGERTER FREISETZUNG ZUR ORALEN VERABREICHUNG SOWIE DEREN HERSTELLUNGSVERFAHREN
SLOW RELEASE PHARMACEUTICAL COMPOSITIONS TO BE ORALLY ADMINISTERED, AND METHOD FOR PREPARING SAME

(30) Priorité: 17.05.1990 BE 9000524
(43) Date de publication de la demande: 06.05.1992
(73) Titulaire: EUROPHARMACEUTICALS S.A., B-1180 Uccle (BE)
(72) Inventeur: Remol, Jean Pol, B-9000 Gent (BE)
(74) Mandataire: Schmitz, Yvon
(86) Numéro de dépôt international: EP9100919
(87) Numéro de publication internationale: WO9117743

(56) Documents cités:
- Drug Development and Industrial Pharmacy, vol. 13, No. 8, 1987, Marcel Dekker, Inc. (New York, US), N.A. Shaikh et al.: "Evaluation of ethylcellulose as a matrix for porlonged release formulations I. Water soluble drugs: acetaminophen and theophylline", pp. 1345-1369, see abstract; p. 1347, lines 18-25; p. 1348, lines 1-14

## Description

La présente invention est relative à des compositions pharmaceutiques à administrer par voie orale et dont la ou les substances actives se libèrent de façon lente, régulière et stable ainsi qu'à leur procédé de préparation.

L'intérêt qui se manifeste actuellement en thérapeutique vis-à-vis de ces formes pharmaceutiques dites "à libération prolongée" provient des avantages qu'elle présente par rapport aux formes conventionnelles, lesquelles libèrent leur substance de façon plus rapide ou plus brutale.

Parmi ces avantages, on citera, notamment :
- leur aptitude à produire dans l'organisme des concentrations en principe actif plus uniformes dans le temps;
- la possibilité qu'elles offrent d'assurer une imprégnation médicamenteuse suffisante à l'organisme pendant les périodes où les prises de médicament sont irrégulières voir absentes, par exemple pendant les périodes nocturnes et pour les médicaments à courte durée d'action;
- la diminution des variations interindividuelles des taux sanguins en médicament, obtenues par la prolongation et la régularisation de la résorption;
- la réduction du nombre de prises journalières, ce qui simplifie la posologie et entraîne une meilleure observance du patient vis-à-vis de cette posologie;
- la possibilité d'utiliser des doses élevées du médicament tout en minimisant le risque d'atteindre les concentrations toxiques, en particulier dans les cas de médicaments dont l'indice thérapeutique est plus élevé;
- la possibilité d'éviter certains effets secondaires indésirables provoqués par certains médicaments, comme des effets digestifs, tels que nausées, vomissements et intolérances gastriques.

Actuellement, les types de compositions les plus utilisées dans le domaine de la libération prolongée de principes actifs sont les comprimés et les microgranules.

A cet égard, l'article dans Drug Development And Medical Industrial Pharmacy, Vol. 13, n° 8, 1987 de N.A. Shaikh et coll., "Evaluation of ethylcellulose as a matrix for prolonged release formulations. I. Water soluble drugs : acetaminophen and theophylline", p. 1345-1369, décrit des compositions pharmaceutiques à base de granulés du type à libération prolongée destinées à l'administration par voie orale comprenant une substance pharmacologiquement active et un excipient pharmacologiquement acceptable dont l'excipient comprend de l'éthylcellulose.

La présente invention a pour but de prévoir une composition pharmaceutique à base de granulés à libération prolongée destinée à l'administration par voie orale comprenant une substance pharmacologiquement active et un excipient pharmacologiquement acceptable assurant une libération plus contrôlée, c'est-à-dire plus lente, régulière et stable de la substance pharmacologiquement active après administration, que les compositions pharmaceutiques du même type connues jusqu'à présent.

A cet effet, suivant l'invention, la substance pharmacologiquement active est de la pentoxifylline et l'excipient comprend au moins un mélange d'une éthylcellulose d'une viscosité de 20 à 25 mPa.s et d'une éthylcellulose d'une viscosité de 280 à 320 mPa.s, la substance active étant granulée avec une solution alcoolique de l'éthylcellulose de viscosité de 20 à 25 mPa.s, le rapport en poids de l'éthylcellulose de viscosité de 20 à 25 mPa.s à l'éthylcellulose de viscosité de 280 à 320 mPa.s étant de 1/5 à 1/20.

Avantageusement, les éthylcelluloses précitées ont respectivement des viscosités de 22 mPa.s et 300 mPa.s, le rapport en poids de l'éthylcellulose de viscosité de 22 mPa.s à celle de viscosité de 300 mPa.s est de 1/10 et la solution alcoolique précitée contient 10 % d'éthylcellulose de viscosité de 22 mPa.s.

Suivant une forme de réalisation particulièrement avantageuse de l'invention, l'excipient comprend au moins une substance choisie dans le groupe comprenant le stéarate de magnésium, le talc, l'amidon, l'acide stéarique et le dioxyde de silicium, le stéarate de magnésium convenant particulièrement bien à cet effet.

L'invention se rapporte également à la préparation de ces compositions pharmaceutiques à libération prolongée.

Ainsi qu'on l'a déjà précisé, la composition pharmaceutique de la présente invention est constituée de pentoxifylline comme substance pharmacologiquement active et d'un excipient pharmacologiquement acceptable formé d'un mélange de deux éthylcelluloses, l'une de faible viscosité, de préférence d'une viscosité de 20 à 25 mPa.s et l'autre de viscosité élevée, de préférence d'une viscosité de 280 à 320 mPa.s. Dans la formulation, les deux éthylcelluloses sont en fait utilisées pour obtenir une libération prolongée de la pentoxifylline. En fait, la pentoxifylline se libère par diffusion de la matrice d'éthylcelluloses. Cette matrice se compose d'une part d'un granulé de substance active à base d'éthylcellulose de faible viscosité (poids moléculaire peu élevé) comme liant et d'autre part d'éthylcellulose de viscosité élevée (poids moléculaire élevé). On utilise donc deux types d'éthylcelluloses; une éthylcellulose dont la viscosité est faible, c'est-à-dire de 20 à 25 mPa.s et une éthylcellulose dont la viscosité est élevée, c'est-à-dire de 280 à 320 mPa.s. A cet égard, on notera que les valeurs des viscosités données dans le cadre de la présente invention sont mesurées dans des conditions ambiantes, c'est-à-dire à la pression atmosphérique et à la température ambiante. Le choix de ces deux éthylcelluloses de viscosités différentes et leur mélange dans des rapports pondéraux déterminés allant de 1/5 à 1/20 permet ainsi un contrôle parfait, c'est-à-dire une libération lente, régulière et stable de la pentoxifylline. Pour obtenir des granulés de substance active, il est nécessaire de mélanger celle-ci avec une solution alcoolique de l'éthylcellulose de faible viscosité. En effet, l'utilisation d'éthylcellulose sous forme de poudre (donc pas comme liant en solution alcoolique) donnerait une libération trop rapide de la substance active.

Les compositions pharmaceutiques à base de granulés à libération prolongée de l'invention se présentent avantageusement sous la forme de dose unitaire ou posologique contenant la pentoxifylline comme substance active et, comme excipient, un mélange d'éthylcelluloses additionné, par exemple, d'un lubrifiant, tel que le stéarate de magnésium. On pourrait bien entendu utiliser à la place du stéarate de magnésium. d'autres excipients, tels que le talc, l'amidon (de maïs, de pommes de terre. de froment, etc.), l'acide stéarique, le dioxyde de silicium. Les expressions "dose unitaire" et "dose posologique" telles qu'utilisées dans le présent mémoire, désignent une dose physiquement déterminée contenant une quantité déterminée de pentoxifylline, cette quantité étant telle qu'une ou plusieurs doses sont nécessaires pour obtenir un effet avantageux. D'une façon générale, ces compositions se présentent sous la forme de comprimé, gélule, sachet ou cachet, dont la dose en pentoxifylline a été préétablie de sorte que l'administration de la dose journalière de la pentoxifylline pourra se faire d'une façon très simple. Ces comprimés, gélules, sachets ou cachets peuvent contenir, par exemple, jusqu'à 800 mg de pentoxifylline.

Un autre avantage essentiel des compositions ou formes posologiques unitaires de l'invention consiste dans la possibilité de réduire fortement les quantités d'excipient autres que les éthylcelluloses comparativement aux formulations du même type connues jusqu'à présent.

On donne ci-après des exemples non limitatifs de formulations galéniques de l'invention destinées à l'administration par voie orale.

### Exemple 1

Préparation de 250.000 comprimés de la formulation :

| | |
|---|---|
| pentoxifylline | 400,0 mg |
| éthylcellulose 22 mPa.s | 20,0 mg |
| éthylcellulose 300 mPa.s | 200,0 mg |
| stéarate de magnésium | 3,0 mg |

Dans un mélangeur d'une capacité de 300 kg, on introduit 100 kg de pentoxifylline. On prépare une solution alcoolique à 10% en poids/volume d'éthylcellulose de 22 mPa.s en dissolvant 5 kg d'éthylcellulose (22 mPa.s) dans 50 litres d'éthanol dénaturé (par l'éther). La dissolution s'effectue à froid dans un mélangeur. On granule la pentoxifylline avec la solution alcoolique d'éthylcellulose 22 mPa.s. On tamise le granulat humide sur un tamis en acier inoxydable de 840 µm et on sèche ensuite le granulat tamisé dans une étuve à 60°C. Une fois séché, le granulé est passé sur tamis en acier inoxydable de 750 µm et le granulé tamisé est alors mélangé dans un mélangeur avec 50 kg d'éthylcellulose de viscosité de 300 mPa.s. Enfin, on ajoute 0,75 kg de stéarate de magnésium et on homogénéise pendant 3 minutes. Les comprimés sont réalisés sur une machine rotative Courtoy de type R 100, munie des poinçons adéquats.

La composition de la formulation précitée a été sélectionné expérimentalement avec l'objectif d'obtenir une libération in vitro de la pentoxifylline de près de 80 % après 8 heures. On a constaté à cet égard que l'utilisation d'éthylcellulose 22 mPa.s sous forme de poudre (donc pas comme liant en solution alcoolique) donnait une libération trop rapide de la pentoxifylline (90 % après 6 heures) et que l'utilisation d'une solution à 5 % d'éthylcellulose 22 mPa.s produisait également une libération trop rapide de la pentoxifylline (100 % après 6 heures). Le choix s'est donc porté sur une solution alcoolique à 10 % d'éthylcellulose 22 mPa.s comme liant. En fait, le choix de ces deux types d'éthylcelluloses et leur mélange dans le rapport pondéral de 1/10 (éthylcellulose 22 mPa.s/éthylcellulose 300 mPa.s) permet un contrôle parfait du taux de libération de la pentoxifylline. Le stéarate de magnésium est quant à lui ajouté comme lubrifiant et n'a donc aucune influence sur la vitesse de libération du principe actif.

Encore un autre avantage de la matrice d'éthylcelluloses comparativement aux matrices habituellement utilisées pour la réalisation de comprimés est qu'elle est assez insensible aux fluctuations de pression et de compression.

### Exemple 2

Formulation contenant 600 mg de pentoxifylline par comprimé :

| | |
|---|---|
| pentoxifylline | 600 mg |
| éthylcellulose 22 mPa.s | 30 mg |
| éthylcellulose 300 mPa.s | 300 mg |
| stéarate de magnésium | 4,5 mg |

### Exemple 3

Formulation contenant 800 mg de pentoxifylline par comprimé :

| | |
|---|---|
| pentoxifylline | 800 mg |
| éthylcellulose 22 mPa.s | 40 mg |
| éthylcellulose 300 mPa.s | 400 mg |
| stéarate de magnésium | 6 mg |

On notera qu'outre les excellents résultats obtenus des analyses effectuées démontrant clairement la libération prolongée de la pentoxifylline (libération in vitro de près de 80 % après 8 heures), les études de stabilité effectuées ont permis de conclure à une excellente stabilité de la pentoxifylline après 6 mois de conservation à une température de 40°C.

## Revendications

1. Composition pharmaceutique à base de granulés du type à libération prolongée destinée à l'administration par voie orale comprenant une substance pharmacologiquement active et un excipient pharmacologiquement acceptable assurant une libération lente, régulière et stable de la substance pharmacologiquement active après administration, caractérisée en ce que la substance pharmacologiquement active est de la pentoxifylline et l'excipient comprend au moins un mélange d'une éthylcellulose d'une viscosité de 20 à 25 mPa.s et d'une éthylcellulose d'une viscosité de 280 à 320 mPa.s, la substance active étant granulée avec une solution alcoolique de l'éthylcellulose de viscosité de 20 à 25 mPa.s, le rapport en poids de l'éthylcellulose de viscosité de 20 à 25 mPa.s à l'éthylcellulose de viscosité de 280 à 320 mPa.s étant de 1/5 à 1/20.

2. Composition pharmaceutique suivant la revendication 1, caractérisée en ce que les éthylcelluloses précitées ont respectivement des viscosités de 22 mPa.s et 300 mPa.s, le rapport en poids de l'éthylcellulose de viscosité de 22 mPa.s à celle de viscosité de 300 mPa.s est de 1/10 et la solution alcoolique précitée contient 10 % d'éthylcellulose de viscosité de 22 mPa.s.

3. Composition pharmaceutique suivant l'une ou l'autre des revendications 1 et 2, caractérisée en ce que l'excipient comprend au moins une substance choisie dans le groupe comprenant le stéarate de magnésium, le talc, l'amidon, l'acide stéarique et le dioxyde de silicium.

4. Composition pharmaceutique suivant la revendication 3, caractérisée en ce que la substance précitée est le stéarate de magnésium.

5. Composition pharmaceutique suivant l'une quelconque des revendications 1 à 4, caractérisée en ce qu'elle se présente sous la forme d'une dose unitaire ou posologique.

6. Composition pharmaceutique suivant la revendication 5, caractérisée en ce qu'elle est un comprimé, une gélule, un sachet ou un cachet contenant jusqu' à 800 mg de substance active.

7. Procédé de préparation de la composition pharmaceutique suivant l'une quelconque des revendications 1 à 6, caractérisé en ce qu'on granule la pentoxifylline avec la solution alcoolique d'éthylcellulose de viscosité de 20 à 25 mPa.s, on tamise le granulat humide ainsi obtenu, on le sèche, on tamise les granulés secs que l'on mélange avec l'éthylcellulose de viscosité de 280 à 320 mPa.s, on ajoute le ou les autres excipients et on donne au mélange une forme appropriée à son administration par voie orale, telle que celle de comprimés, gélules, sachets ou cachets, la dose unitaire en substance active de ladite composition pouvant atteindre 800 mg.

## Claims

1. A granule-based pharmaceutical composition of the prolonged release type intended to be administered by mouth, comprising a pharmacologically active substance and a pharmacologically acceptable excipient ensuring slow, regular and stable release of the pharmacologically active substance after administration, characterised in that the pharmacologically active substance is pentoxifylline and the excipient comprises at least a mixture of an ethyl cellulose with a viscosity of 20 to 25 mPa.s and an ethyl cellulose having a viscosity of 280 to 320 mPa.s, the active substance being granulated with an alcohol solution of ethyl cellulose with a viscosity of 20 to 25 mPa.s, the ratio by weight of the ethyl cellulose of 20 to 25 mPa.s viscosity to the ethyl cellulose of 280 to 320 mPa.s viscosity being from 1:5 to 1:20.

2. A pharmaceutical composition according to Claim 1, characterised in that the aforesaid ethyl celluloses respectively have viscosity levels of 22 mPa.s and 300 mPa.s, the ratio by weight of the ethyl cellulose of 22 mPa.s viscosity to that of 300 mPa.s viscosity is 1:10 and the aforesaid alcohol solution contains 10% ethyl cellulose with a viscosity of 22 mPa.s.

3. A pharmaceutical composition according to one or other of Claims 1 and 2, characterised in that the excipient comprises at least one substance chosen from the group comprising magnesium stearate, talc, starch, stearic acid and silicon dioxide.

4. A pharmaceutical composition according to Claim 3, characterised in that the aforesaid substance is magnesium stearate.

5. A pharmaceutical composition according to any one of Claims 1 to 4, characterised in that it is in the form of a unitary or posological dose.

6. A pharmaceutical composition according to Claim 5, characterised in that it is a tablet, a capsule, a sachet or a wafer containing up to 800 mg active substance.

7. A method of producing the pharmaceutical composition according to any one of Claims 1 to 6, characterised in that the pentoxifylline is granulated with the alcohol solution of ethyl cellulose of 20 to 25 mPa.s viscosity, the moist granulate thus obtained is screened, dried, the dry granules are screened and mixed with the ethyl cellulose of 280 to 320 mPa.s viscosity, the other excipient or excipients is or are added and the mixture is given a form appropriate to its administration by mouth, such as that of tablets, capsules, sachets or wafers, the unitary dose of active substance in the said composition being as high as 800 mg.

## Patentansprüche

1. Pharmazeutische Zusammensetzung auf Granulatbasis mit verzögerter Freisetzung zur oralen Verabreichung mit einer pharmakologisch aktiven Substanz und einem pharmakologisch akzeptablen Bindemittel, das eine langsame, gleichmäßige und stabile Freisetzung der pharmakologisch aktiven Substanz nach Verabreichung sicherstellt,
**dadurch gekennzeichnet,**
daß die pharmakologisch aktive Substanz Pentoxyfyllin ist und das Bindemittel wenigstens ein Gemisch aus einer Äthylzellulose mit einer Viskosität von 20 bis 25 mPa.s und einer Äthylzellulose mit einer Viskosität von 280 bis 320 mPa.s umfaßt, wobei die aktive Substanz mit einer alkoholischen Lösung der Äthylzellulose mit der Viskosität von 20 bis 25 mPa.s. granuliert und das Gewichtsverhältnis der Äthylzellulose mit der Viskosität von 20 bis 25 mPa.s zur Äthylzellulose mit der Viskosität von 280 bis 320 mPa.s 1/5 bis 1/20 ist.

2. Pharmazeutische Zusammensetzung nach Anspruch 1, dadurch gekennzeichnet, daß die vorgenannten Äthylzellulosen Viskositäten von 22 mPa.s bzw. 300 mPa.s haben, das Gewichtsverhältnis der Äthylzellulose mit der Viskosität von 22 mPa.s zu derjenigen mit der Viskosität von 300 mPa.s 1/10 ist, und die vorgenannte alkoholische Lösung 10% Äthylzellulose mit der Viskosität von 22 mPa.s enthält.

3. Pharmazeutische Zusammensetzung nach dem einen oder anderen der Ansprüche 1 und 2, dadurch gekennzeichnet, daß das Bindemittel wenigstens eine Substanz umfaßt, die aus der Gruppe ausgewählt ist, welche Magnesiumstearat, Talkum, Stärke, Stearinsäure und Siliziumdioxyd umfaßt.

4. Pharmazeutische Zusammensetzung nach Anspruch 3, dadurch gekennzeichnet, daß die vorgenannte Substanz Magnesiumstearat ist.

5. Pharmazeutische Zusammensetzung nach irgendeinem der Ansprüche 1 bis 4, dadurch gekennzeichnet, daß sie sich in Form einer unitären oder posologischen Dosis darbietet.

6. Pharmazeutische Zusammensetzung nach Anspruch 5, dadurch gekennzeichnet, daß sie eine Tablette, eine Kapsel, ein Beutelchen oder eine Oblate ist und bis zu 800 mg an aktiver Substanz enthält.

7. Verfahren zum Herstellen der pharmazeutischen Zusammensetzung nach irgendeinem der Ansprüche 1 bis 6, dadurch gekennzeichnet, daß man das Pentoxyfyllin mit der alkoholischen Lösung der Äthylzellulose mit der Viskosität von 20 bis 25 mPa.s granuliert, das so erhaltene feuchte Granulat absiebt und trocknet, das trockene Granulat absiebt, mit Äthylzellulose mit der Viskosität von 280 bis 320 mPa.s vermischt, das oder die anderen Bindemittel zugibt und dem Gemisch eine zu seiner oralen Verabreichung geeignete Form gibt, beispielsweise diejenige von Tabletten, Kapseln, Beutelchen oder Oblaten, wobei die Einheitsdosis an aktiver Substanz der genannten Zusammensetzung 800 mg erreichen kann.
